# EUROPEAN PATENT SPECIFICATION

(11) **EP 2 830 679 B1**
(45) Date of publication and mention of the grant of the patent: **14.08.2019**
(21) Application number: 13716886.0
(22) Date of filing: 14.03.2013
(51) Int. Cl.: A61M 5/28, A61M 5/158, A61M 5/24, A61M 5/172, A61J 1/14, A61M 5/142, A61M 5/145, A61M 5/31

(54) **MOTION ACTIVATED MECHANISMS FOR A DRUG DELIVERY DEVICE**
BEWEGUNGSAKTIVIERTE MECHANISMEN FÜR EINE WIRKSTOFFFREISETZUNGSVORRICHTUNG
MÉCANISMES ACTIONNÉS PAR MOUVEMENT DESTINÉS À UN DISPOSITIF D'ADMINISTRATION DE MÉDICAMENT

(30) Priority: 26.03.2012 US 201213429942
(43) Date of publication of application: 04.02.2015
(73) Proprietor: Medimop Medical Projects Ltd., 43665 Ra'anana (IL)
(72) Inventor: CABIRI, Oz, 71799 Macabim-reut (IL)
(74) Representative: Patentanwälte Vollmann Hemmer Lindfeld Partnerschaft mbB
(86) International application number: PCT/US2013/031598
(87) International publication number: WO 2013/148270

(56) References cited:
- WO-A1-97/21457
- WO-A2-2008/024810
- WO-A2-2008/024814
- US-A1- 2003 199 825
- US-A1- 2007 167 912
- US-A1- 2008 051 727

## Description

### RELATED APPLICATIONS

This application claims the benefit of priority of US pending patent application, serial number 13/429,942, filed on March 26, 2012.

### FIELD AND BACKGROUND OF THE INVENTION

The present invention, in some embodiments thereof, relates to an apparatus wearable by a recipient and method for delivering a substance to a recipient, more particularly, but not exclusively, to an apparatus with a mechanical drive train for priming the apparatus, for example by unsealing a reservoir containing a drug and/or by locking a door and subsequently delivering the drug to a recipient.

US published patent application 2009/0093792 to the present author discloses an apparatus for administering a substance to a subject. A vial contains the substance and a stopper is disposed within the vial and is slidably coupled to the vial. A first threaded element is (a) rotatable with respect to the vial and (b) substantially immobile proximally with respect to the vial during rotation of the first threaded element. A second threaded element is threadably coupled to the first threaded element. At least a distal end of the second threaded element is substantially non-rotatable with respect to the vial, and the distal end of the second threaded element defines a coupling portion that couples the second threaded element to the stopper. The first threaded element, by rotating, linearly advances the stopper and at least the distal end of the second threaded element toward a distal end of the vial. A vial piercing mechanism is movably (e.g., rotatably) coupled to a housing base. As part of the insertion of vial into the housing base, a seal at distal end of the vial is pierced by pressing the seal against the piercing mechanism. The substance is configured to subsequently flow through a tube toward an activation mechanism, which is typically coupled to the housing base, and is configured to insert a cannula and/or a needle through the subject's skin and to deliver the substance via the cannula and/or the needle.

US patent no. 5,858,001 to Tsals discloses a liquid drug delivery device adapted to be adhered to the skin of a subject by a base member defining a skin-contacting surface having an adhesive coating. A columnar cartridge serves as reservoir for the drug and is incorporated in a housing, which is connected to the base member such that in use the longitudinal axis of the cartridge is disposed substantially parallel to the skin-contacting surface. A delivery needle communicating in use with the interior of the cartridge penetrates the skin of the subject when the housing snaps downward relative to the base member. This action also causes the actuation of a citric acid/sodium bicarbonate gas generator which generates a gas to move a piston within the cartridge, compressing the drug compartment. This compression causes a stopper to be penetrated by a conduit in communication with the delivery needle, allowing the drug to be ejected from the compartment through the needle and into the subcutaneous tissue of the subject.

US published patent application 2006/0173408 to Wyrick discloses a reloadable medicine injector and methods in which a barrel with a receiving cavity is adapted to slidably to receive a syringe subassembly for axial movement therein. Upon removal of a safety and release of a syringe driver, the syringe driver moves forward and injects the syringe needle. A plurality of penetration controls are shown for controlling injection needle penetration depth. In some embodiments, the injector makes use of a double needle assembly in which a double needle hub mounts a seal penetration needle that projects rearwardly toward a penetrable seal on the associated ampule. A flesh penetration needle projects forwardly. In practice, both needles can be made integral.

US patent no. 5,997,501 to Gross discloses an intradermal drug delivery device comprising a housing having a drug reservoir therewithin. A microprocessor-controlled electrolytic cell provides gas to expand a gas generation chamber and thereby contract the reservoir. A hollow needle, communicating at an inner end thereof with the reservoir, extends from a lower surface of the housing such that contraction of the reservoir forces drug to escape therefrom via the needle. The device permits delivery of drugs of relatively large molecular weights at slow rates.

US published patent application 2011/0178472 and PCT application WO 2011/090955 to the present author disclose a needle assembly adapted for fluid communication with a cartridge containing a substance to be delivered to a subject. The needle assembly characterized by a biasing device arranged to apply a biasing force on a needle to cause the needle to protrude outwards of a housing to pierce the subject, and biasing device release apparatus including a biasing device arrestor that initially blocks movement of the biasing device. After finishing the drug administration, the needle release apparatus lifted off the patient's body, which causes the safety latch to move back to the down position and the needle to be retracted back into the housing.

A safety latch position sensor is provided for sensing when safety latch moves to an up position indicating that the device has been attached to a patient. A controller initiates operation of an actuator after a predetermined time delay (e.g., 5-15 seconds) to ensure that the drug delivery apparatus was indeed placed on purpose on the patient for delivering the drug. When operated, the actuator rotates a shaft causing the biasing device arrestor to move linearly out of an aperture. As soon as the biasing device arrestor has moved out of the aperture, the biasing device is no longer blocked and it now pushes down on a needle piercing the patient's skin.

Additional background art includes US published patent applications 2011/0178472 to the same author, US patent no. 7,789,862 to Thorne, patent no. 7,780,636 to Radmer, US patent no. 5957895 to Sage, US patent no. 7,918,843 to Genosar, and US patent no. 7,789,862 to Thorne.

### SUMMARY OF THE INVENTION

The invention is defined in claim 1.

According to an aspect of some embodiments of the present invention there is provided an apparatus wearable by a recipient having a single motion drive train for sequentially unsealing a reservoir and delivering a drug to the recipient. The apparatus may include a seal of the reservoir. The apparatus may also include a controlled rate power source. The apparatus may also include a plunger driven by the controlled rate power source. The apparatus may also include a hollow needle. An initial movement of the plunger may drive the hollow needle through the seal and subsequent movement of the plunger may discharge the drug from the reservoir.

According to some embodiments of the invention, the controlled rate power source may include a direct current motor, a stepper motor and/or a linear actuator.
According to some embodiments of the invention, the apparatus may further include a surface configured for attaching to a skin of the recipient.

According to some embodiments of the invention, the apparatus may further include a pathway for movement of the reservoir. The initial movement of the plunger may impel the reservoir along the pathway.

According to some embodiments of the invention, the pathway may be substantially parallel to the surface.

According to some embodiments of the invention, the plunger may be configured for discharging a liquid having a viscosity of at least 10 centipoise.

According to some embodiments of the invention, the plunger may be configured for discharging at a substantially constant rate for at least 1 minute.

According to some embodiments of the invention, the reservoir may be configured for long term storage of the drug inside of the apparatus.

According to some embodiments of the invention, the apparatus may further include a hypodermic needle and a needle release for inserting the hypodermic needle into the recipient. The needle release may be synchronized to the plunger.

According to some embodiments of the invention, a direction of the needle release may be non-parallel to a direction of the movement of the plunger.

According to some embodiments of the invention, a direction of the needle release may be substantially orthogonal to a direction of the movement of the plunger.

According to some embodiments of the invention, the apparatus may further include a processor for controlling the controlled rate power source.

According to some embodiments of the invention, the apparatus may further include a septum located in a needle opening of the apparatus. Upon release of a hypodermic needle, the hypodermic needle may puncture the septum before being inserted into the recipient.

According to some embodiments of the invention, the apparatus may further include a rotation sensor. The controlling may be according to an output of the rotation sensor.

According to some embodiments of the invention, the apparatus may further include a rotation sensor. The processor may sense the puncturing of the seal based on an output of the rotation senor.

According to some embodiments of the invention, the hollow needle may be initially in fluid communication with the reservoir. The seal may be configured for sealing the hollow needle.

According to some embodiments of the invention, the reservoir may include a cartridge insertable into the apparatus.

According to an aspect of some embodiments of the present invention there is provided a method for delivering a drug to a recipient with a delivery apparatus. The drug may be contained in a reservoir initially sealed by a seal for long term aseptic storage. The delivery apparatus may include a hollow needle and a plunger. The method may include attaching a surface of the delivery apparatus to the recipient. The method may also include applying a force with the plunger to contents of the reservoir. The method may also include puncturing the seal with the hollow needle by the force. The method may also include discharging the drug from the reservoir by the force subsequent to the puncturing. The method may also include detecting a rate of the discharging, and the method may also include adjusting the rate of the discharging based on a result of the detecting.

According to some embodiments of the invention, the applying of the force may be in a direction substantially parallel to the surface.

According to some embodiments of the invention, the method may further include supplying a torque, and converting the torque into the force.

According to some embodiments of the invention, the detecting may be by measuring a rotation driven by the torque.

According to some embodiments of the invention, the discharging may be at a substantially constant rate for at least 1 minute.

According to some embodiments of the invention, the method may further include releasing a hypodermic needle into the recipient by the apparatus subsequent to the puncturing.

According to some embodiments of the invention, the method may further include releasing a hypodermic needle by the apparatus into the recipient in a direction non-parallel to the applying of the force.

According to some embodiments of the invention, the method may further include releasing a hypodermic needle by the apparatus into the recipient in a direction substantially orthogonal to the applying of the force.

According to some embodiments of the invention, the method may further include sensing by the delivery apparatus of a puncturing of the seal.

According to an aspect of some embodiments of the present invention, there is provided a method for delivering a drug to a recipient with a delivery apparatus, the apparatus may include an internal space initially sealed by a septum for long term aseptic storage. The delivery apparatus may also include a needle and a processor. The method may include attaching a surface of the delivery apparatus to the recipient. The method may also include puncturing the septum with the needle in response to a command of the processor. The method may also include discharging the drug subsequent to puncturing the septum. The method may also include, detecting a rate of the discharging and adjusting by the processor of the rate of the discharging based on a result of the detecting.

According to some embodiments of the invention, the discharging may be driven by an actuator.

According to some embodiments of the invention, the puncturing may be triggered by the actuator.

According to some embodiments of the invention, the actuator may include a motor. The method may further include transforming a rotational motion of the motor into a linear motion. The discharging may be driven by the linear motion.

According to some embodiments of the invention, the septum may seal a needle opening in a housing of the apparatus.

According to some embodiments of the invention, the needle may include a hypodermic needle. The method may further include inserting the hypodermic needle into the recipient subsequent to the puncturing and prior to the discharging.

According to an aspect of some embodiments of the present invention, there is provided an apparatus wearable by a recipient for delivering a drug to the recipient. The apparatus may include an initially aseptic internal space. The apparatus may also include a septum initially sealing the internal space. The apparatus may also include a controlled rate power source. The apparatus may also include a plunger driven by the controlled rate power source, and a hollow needle. The controlled rate power source may trigger puncturing the septum by the hollow needle, and subsequently, the power source may drive movement of the plunger, discharging the drug from the reservoir.

According to an aspect of some embodiments of the present invention there is provided a method for delivering a drug to a recipient with a drug delivery device. The drug delivery device may include an attaching base and a mechanical drive train and a reservoir containing the drug. The method may include attaching the base to the recipient; driving a mechanical element with a motion of the mechanical drive train to prime the delivery device while attached to the recipient, and subsequently impelling a plunger into the reservoir by continued motion of the drive train, the plunger discharging the drug from the reservoir.

According to some embodiments of the invention, the priming is irreversible. According to some embodiments of the invention, the priming includes unsealing the reservoir. According to some embodiments of the invention, the unsealing includes puncturing a septum with a hollow needle and the discharging is through the hollow needle.

According to some embodiments of the invention, the priming includes locking a door closed at least partially blocking an opening for accessing the reservoir.
According to some embodiments of the invention, the reservoir is contained in a cartridge and the method may further include inserting the cartridge through the opening into the apparatus prior to the locking.

According to some embodiments of the invention, the method may further include sensing a rate of the discharging and adjusting the motion according to a result of the sensing. According to some embodiments of the invention, the motion is substantially parallel to the base. According to some embodiments of the invention, the discharging is at a substantially constant rate for at least 1 minute.

According to some embodiments of the invention, the method may further include inserting a hypodermic needle into the recipient.

According to some embodiments of the invention, a direction of the needle release is non-parallel to the path of motion.

According to some embodiments of the invention, the releasing is in a direction substantially orthogonal to the path of motion.

According to some embodiments of the invention, the method may further include rotating a gear and the rotating may power the motion.

According to an aspect of some embodiments of the present invention there is provided an apparatus for delivering a drug to a recipient. The apparatus may include an attaching base for attachment to the recipient; a mechanical drive train; a reservoir containing the drug; a priming mechanism driven by the motion, and a plunger configured to be impelled into the reservoir by the drive train subsequent to the priming thereby discharging the drug from the reservoir.

According to some embodiments of the invention, the priming is irreversible.

According to some embodiments of the invention, the apparatus may further include a seal initially sealing the reservoir preventing the discharging and a puncturing mechanism configured for being driven through the seal by an initial movement of the drive train.

According to some embodiments of the invention, the puncturing mechanism includes a hollow needle and the discharging is through the hollow needle.

According to some embodiments of the invention, the apparatus may further include an opening for accessing the reservoir; a door having a closed position at least partially blocking the opening, and a locking mechanism driven by the motion to lock the door in the closed position.

According to some embodiments of the invention, the reservoir is included in a cartridge and the cartridge is configured for insertion through the opening into the apparatus.

According to some embodiments of the invention, the apparatus may further include a controlled rate power source driving the drive train.

According to some embodiments of the invention, the controlled rate power source includes at least one element selected from the group consisting of a direct current motor, a stepper motor, and a linear actuator.

According to some embodiments of the invention, the motion is substantially parallel to the base.

According to some embodiments of the invention, the plunger is configured for discharging a liquid having a viscosity of at least 10 centipoise.

According to some embodiments of the invention, plunger is configured for discharging at a substantially constant rate for at least 1 minute.

According to some embodiments of the invention, reservoir is configured for long term storage of the drug inside of the apparatus.

According to some embodiments of the invention, the apparatus may further include a hypodermic needle, and a needle release for inserting the hypodermic needle into the recipient.

According to some embodiments of the invention, the needle release is activated by the motion.

According to some embodiments of the invention, a direction of the needle release is non-parallel path of motion.

According to some embodiments of the invention, a direction of the needle release is substantially orthogonal path of motion.

According to some embodiments of the invention, the apparatus may further include a temporary latch, the temporary latch holding the door in a closed position prior to the locking.

According to some embodiments of the invention, the locking is irreversible.

According to some embodiments of the invention, the locking occurs after a time delay.

According to some embodiments of the invention, the apparatus may further include a septum located in a needle opening of the apparatus such that upon release the hypodermic needle punctures the septum before being inserted into the recipient.

According to some embodiments of the invention, the apparatus may further include a processor for controlling the controlled rate power source.

According to some embodiments of the invention, the apparatus may further include an injection rate sensor and wherein the controlling is according to an output of the injection rate sensor.

According to some embodiments of the invention, the processor is configured for further sensing progress of at least one action selected from the group of the priming and the discharging.

According to some embodiments of the invention, the injection rate sensor includes a rotation sensor.

According to some embodiments of the invention, the hollow needle is initially in fluid communication with the reservoir, and the seal is configured for sealing the hollow needle.

According to some embodiments of the invention, the apparatus may further include a cartridge insertable into the apparatus, the reservoir being included in the cartridge.

According to some embodiments of the invention, the apparatus may further include a lock activated by the drive train.

According to some embodiments of the invention, the base may include an adhesive for the attachment.

According to some embodiments of the invention, the lock includes at least one element selected from the group consisting of a bolt and a pressure activated latch.

According to some embodiments of the invention, the door has a slack and the driving of the locking mechanism includes restraining the slack.

According to an aspect of some embodiments of the present invention there is provided a method for delivering a drug to a recipient with a delivery apparatus, the drug contained in a reservoir initially sealed by a seal for long term aseptic storage, the delivery apparatus including a hollow needle and a plunger. The method may include impelling the plunger into the reservoir via motion of a mechanical drive train; puncturing the seal by driving the hollow needle through the seal via the motion; discharging the drug from the reservoir by via the motion subsequent to the puncturing; detecting a rate of the discharging, and adjusting a rate of the motion based on a result of the detecting.

According to some embodiments of the invention, the method may further include attaching the delivery apparatus to the recipient.

According to some embodiments of the invention, the method may further include delivering the drug into the recipient at an injection site. The motion may be in a direction substantially parallel to a skin surface of the recipient at the injection site.

According to some embodiments of the invention, the method may further include supplying a rotating actuator and converting the rotating into the motion.

According to some embodiments of the invention, the detecting is by measuring the rotation.

According to some embodiments of the invention, the discharging is at a substantially constant rate for at least 1 minute.

According to some embodiments of the invention, the method may further include releasing a hypodermic needle by the delivery apparatus into the recipient subsequent to the puncturing.

According to some embodiments of the invention, the method may further include releasing a hypodermic needle by the delivery apparatus into the recipient in a direction non-parallel to the motion.

According to some embodiments of the invention, the method may further include releasing a hypodermic needle by the delivery apparatus into the recipient in a direction substantially orthogonal to the motion.

According to some embodiments of the invention, the method may further include sensing by the delivery apparatus of the puncturing.

According to some embodiments of the invention, the method may further include activating a locking mechanism of the apparatus by means of the motion.

According to an aspect of some embodiments of the present invention there is provided a method for delivering a drug to a recipient with a delivery apparatus. The delivery apparatus may include an internal space initially sealed by a septum for long term aseptic storage, a needle and a processor. The method may include puncturing the septum with the needle in response to a command of the processor; discharging the drug subsequent to the puncturing; detecting a rate of the discharging, and adjusting by the processor the rate of the discharging based on a result of the detecting.

According to some embodiments of the invention, the discharging is driven by an actuator.

According to some embodiments of the invention, the puncturing is triggered by the actuator.

According to some embodiments of the invention, actuator may further include a motor and the method may further include transforming a rotational motion of the motor into a translational motion, and the discharging may be driven by the translational motion.

According to some embodiments of the invention, the septum seals a needle opening in a housing of the delivery apparatus.

According to some embodiments of the invention, the needle may include a hypodermic needle, and the method may further include inserting the needle into the recipient subsequent to the puncturing and prior to the discharging.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system. For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard disk and/or removable media, for storing instructions and/or data. Optionally, a network connection is provided as well. A display and/or a user input device such as a keyboard or mouse are optionally provided as well.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Figure 1 is a flow chart illustrating an exemplary embodiment of a method for unsealing a reservoir and delivering a drug;
Figure 2a is a perspective external view of an exemplary embodiment of an apparatus for puncturing a septum and delivering a drug;
Figure 2b is a simplified cutaway view of an exemplary embodiment of an apparatus for puncturing a septum and delivering a drug prior to puncturing the septum;
Figure 2c is a simplified cutaway view of the embodiment of Fig. 2b after puncturing the septum;
Figure 2d is a simplified cutaway view of the embodiment of Fig. 2b while delivering the drug;
Figure 3 is a perspective view of the mechanism of another exemplary embodiment of an apparatus for delivering a drug wherein a septum seals a needle prior to puncturing the septum; Figure 3' is a close perspective view of the septum of the embodiment of Fig. 3 prior to puncturing the septum;
Figure 4 is a perspective view of the mechanism the embodiment of Fig. 3 after puncturing the septum;

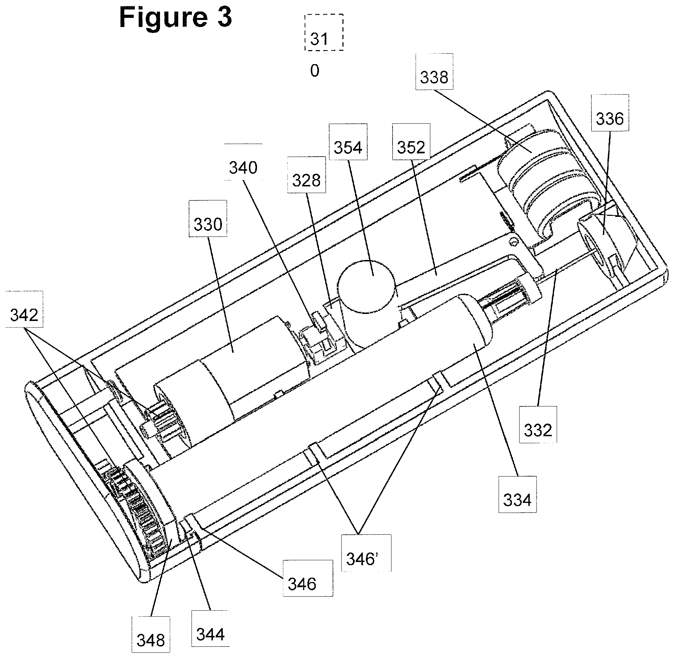

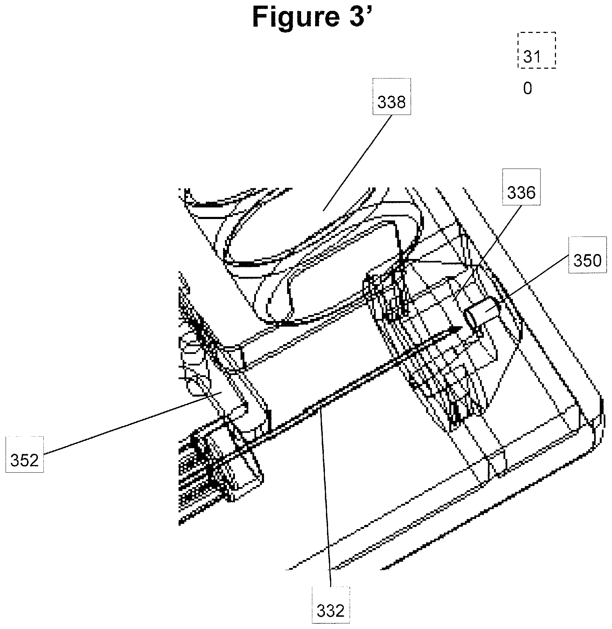

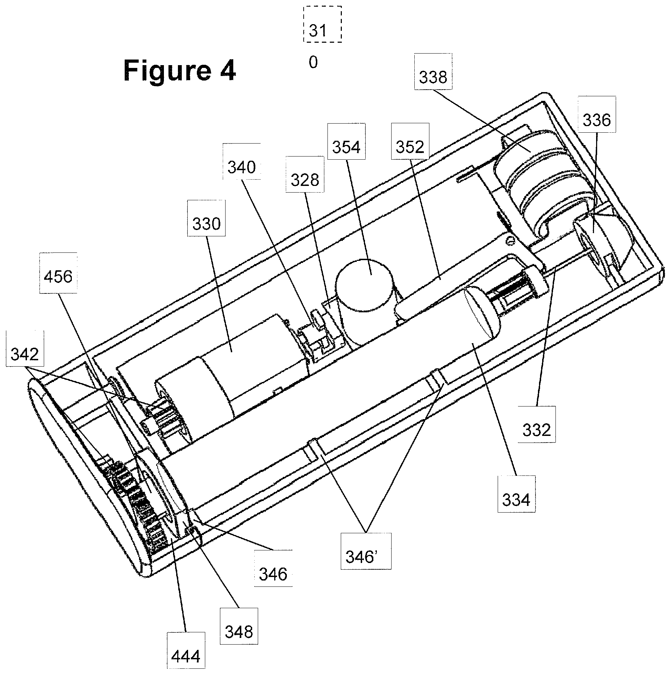

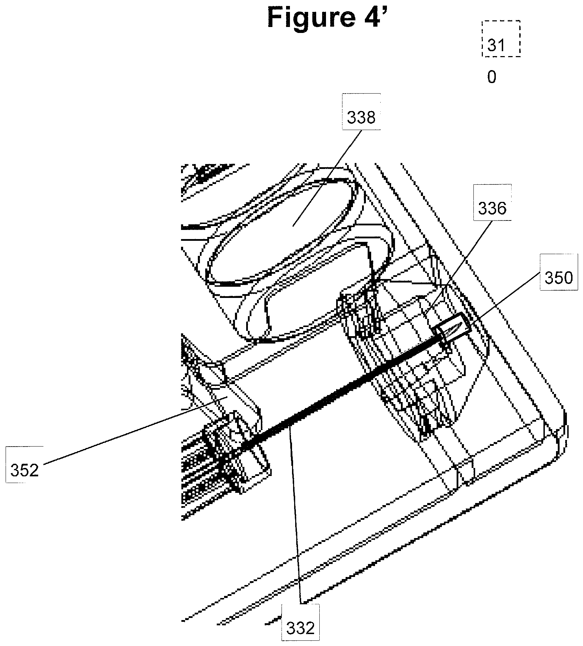

Figure 4' is a close perspective view of the septum of the embodiment of Fig. 3 after puncturing the septum;

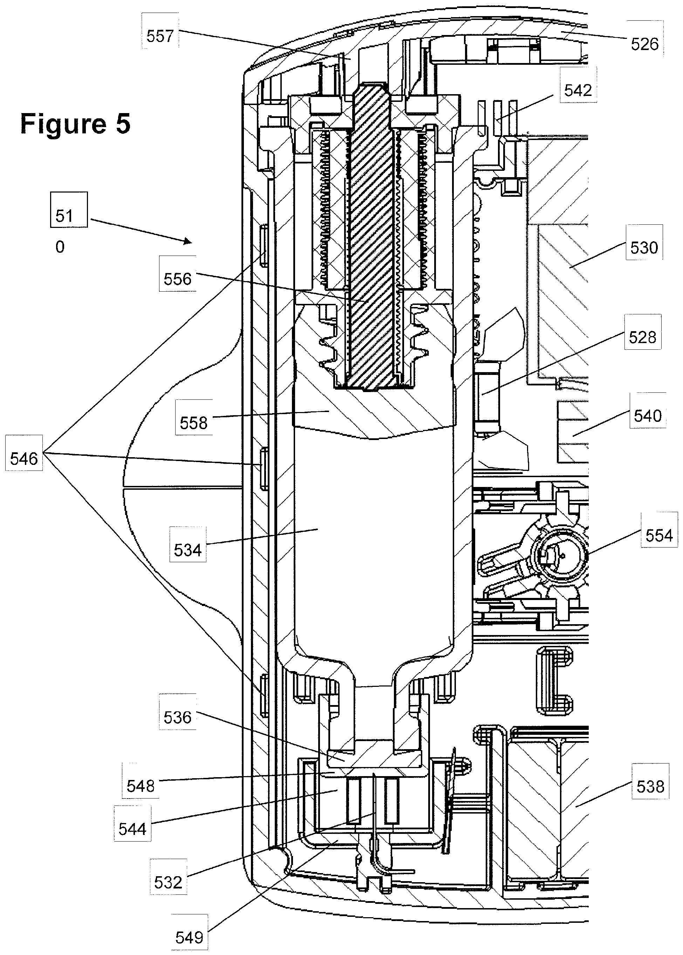

Figure 5 is a cutaway view of a further exemplary embodiment of an apparatus for delivering a drug wherein a septum seals a vial prior to puncturing the septum;

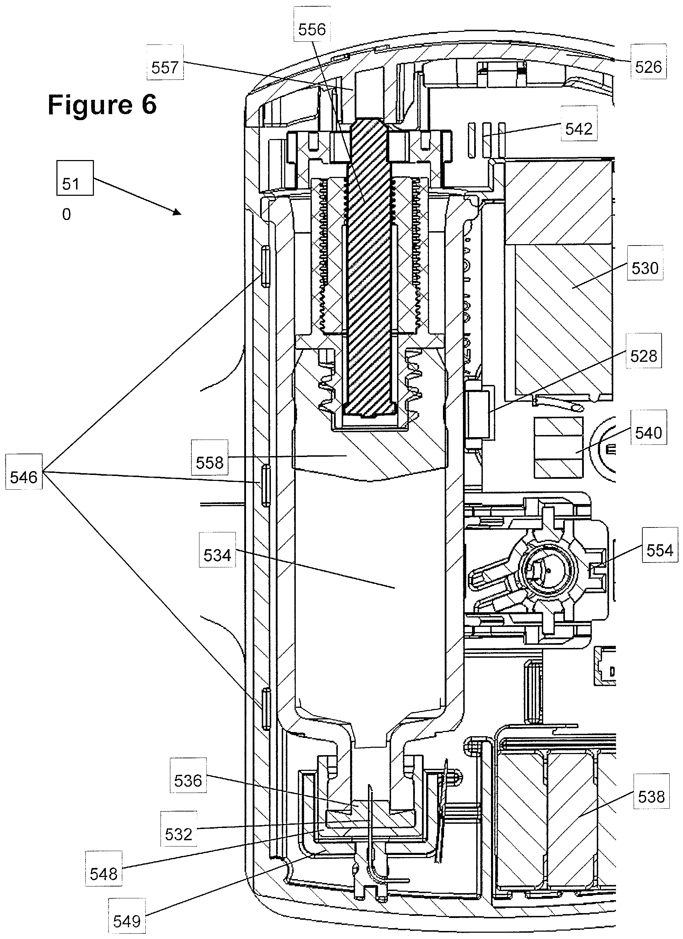

Figure 6 is a cutaway view of the embodiment of Fig. 5 after puncturing the septum;

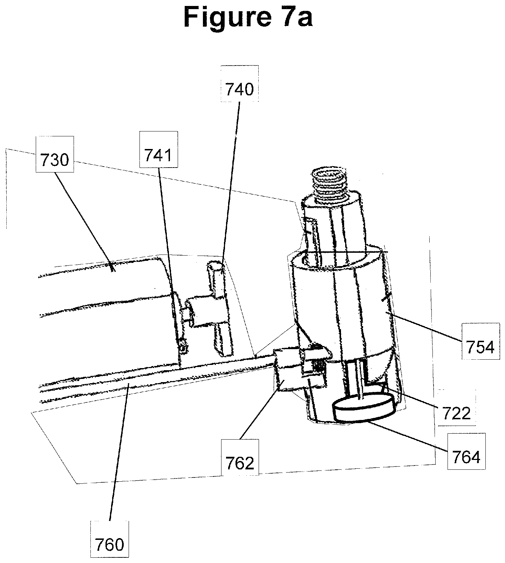

Figure 7a illustrates a perspective view of an exemplary embodiment of a needle insertion mechanism with a septum, prior to releasing the needle;

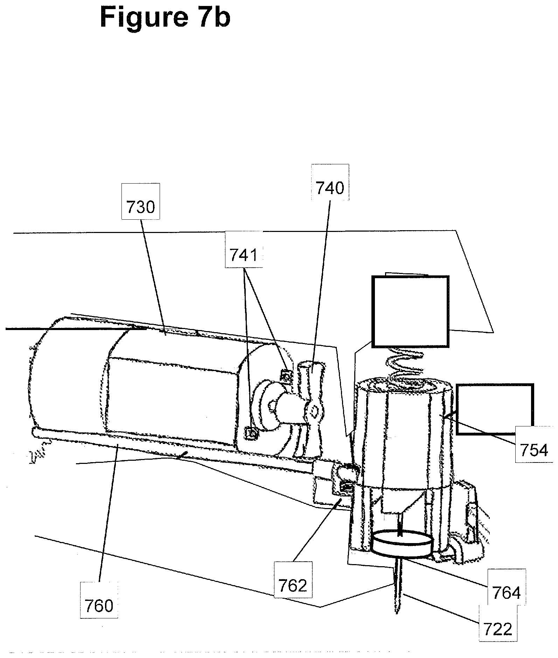

Figure 7b illustrates a perspective view of an exemplary embodiment of a needle insertion mechanism with a septum after releasing of the needle;

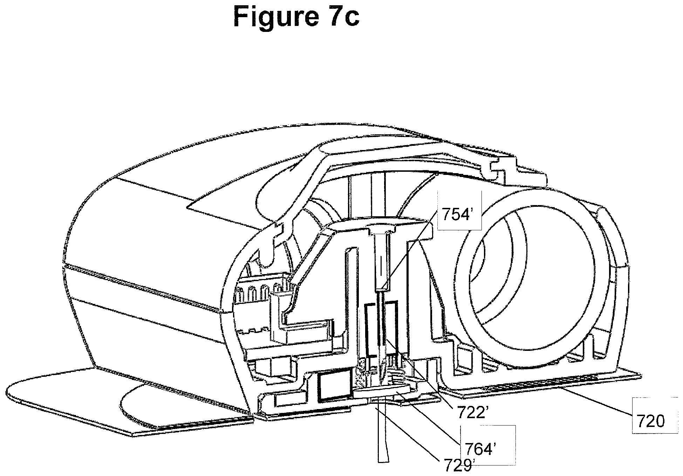

Figure 7c illustrates a cutaway perspective view of an exemplary embodiment of an injection apparatus with a septum and a needle release, previous to releasing the needle;

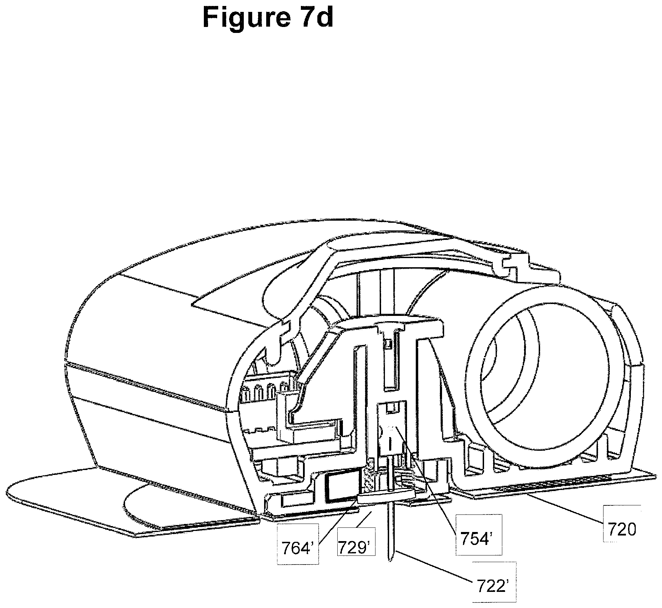

Figure 7d illustrates a cutaway perspective view of an exemplary embodiment of a injection apparatus with a septum and a needle release, after releasing the needle, and

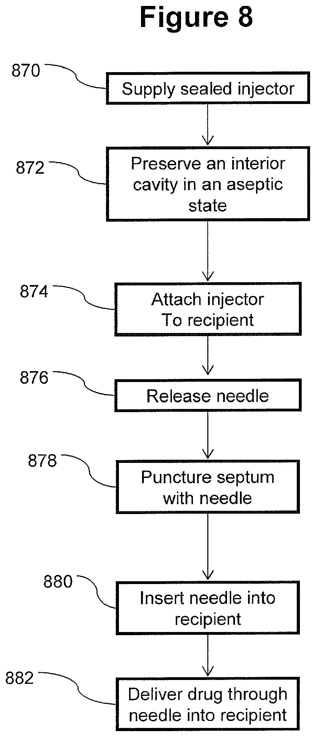

Figure 8 is a flowchart illustrating an exemplary embodiment of a method for delivering a drug to a recipient with an initially sealed injector;

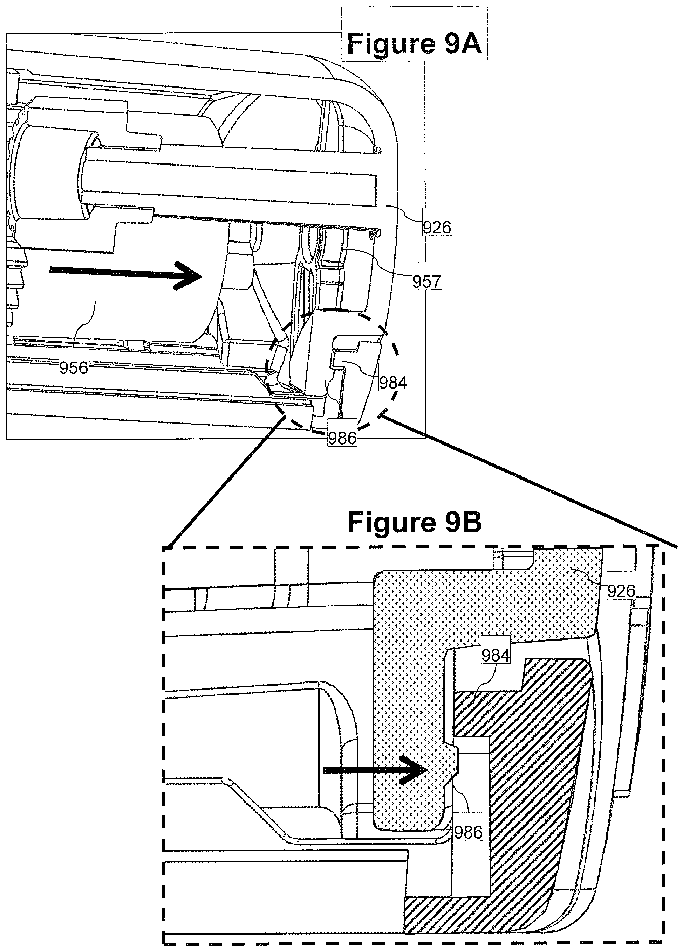

Figures 9A,B illustrate an exemplary pressure activated door latch locking mechanism in accordance with an embodiment of the present invention;

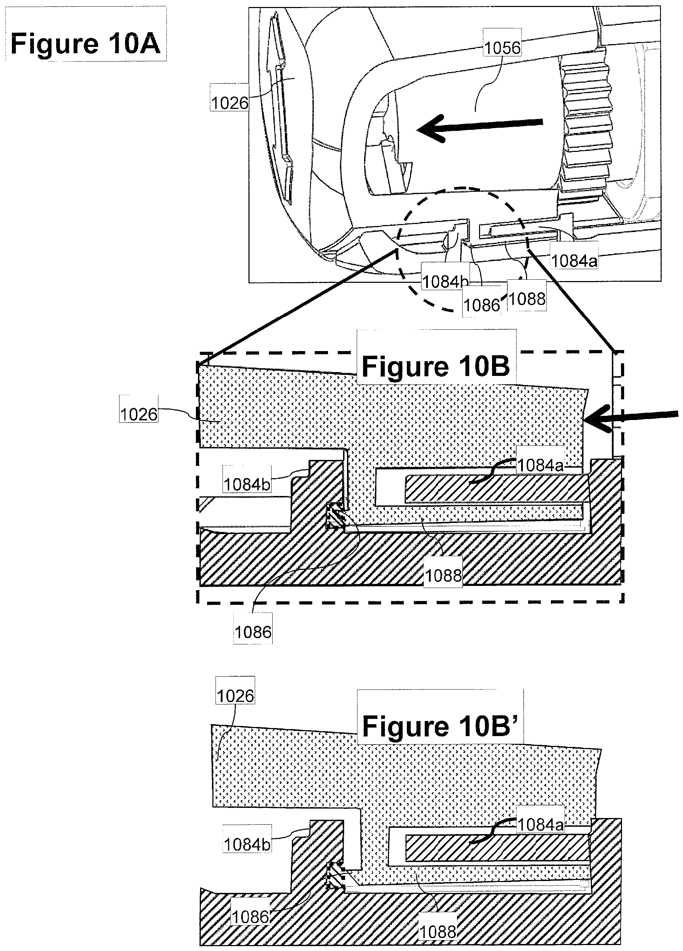

Figures 10A,B,B',C,D,D',D" illustrate an alternative exemplary pressure activated door latch locking mechanism in accordance with an embodiment of the present invention;

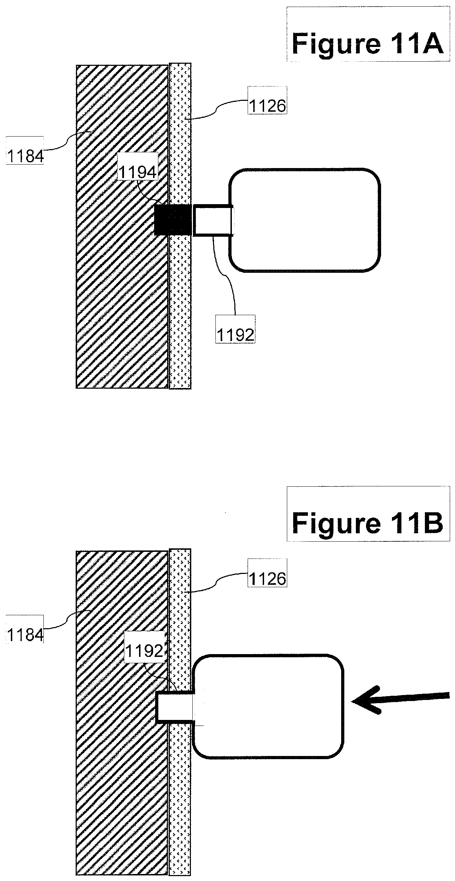

Figures 11A,B illustrate door locking bolt activated by a medicine discharge mechanism in accordance with an embodiment of the present invention; and

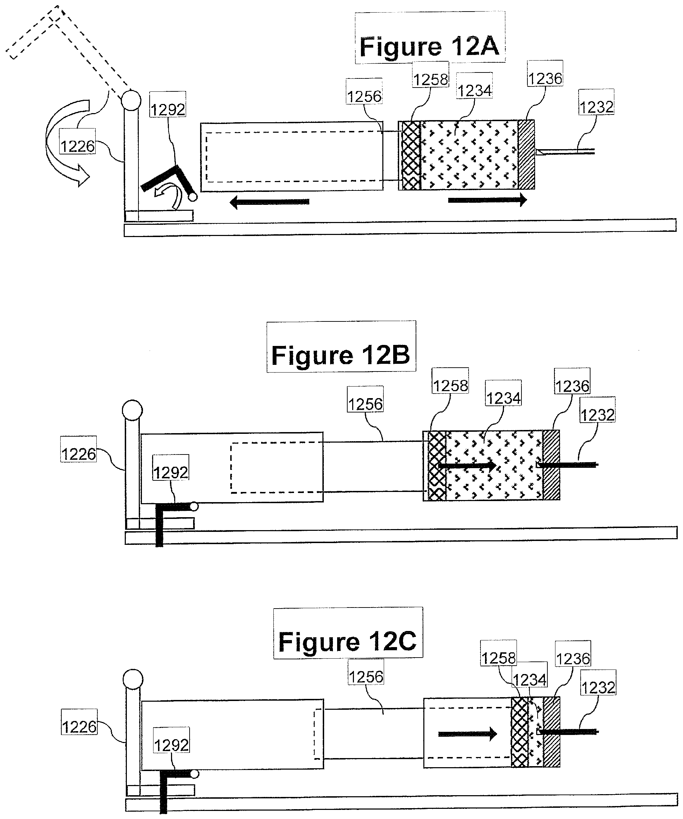

Figures 12A-C illustrate a schematic embodiment of an infuser having a septum puncturing mechanism and a locking mechanism in accordance with an embodiment of the present invention.

### DESCRIPTION OF SPECIFIC EMBODIMENTS OF THE INVENTION OVERVIEW

The present invention, in some embodiments thereof, relates to an apparatus wearable by a recipient and method for delivering a substance to a recipient, more particularly, but not exclusively, to an apparatus with a mechanical drive train for priming the apparatus, for example by unsealing a reservoir containing a drug and/or by locking a door and subsequently delivering the drug to a recipient.

### Drive one Motion and/or Parallel to Base / small light

In some embodiments, an unsealing mechanism and/or a locking mechanism may use the existing drive train of the drug discharge device. For example, the drive train may include a controlled rate power source for example a motor driving a mechanical drive train. For example, the drive train may include a screw assembly that transforms the revolving motion of the motor into a linear motion. For example, the drive train may include a telescoping assembly. In some embodiments, drive train may be unidirectional and/or irreversible, for example, a telescoping assembly may be designed to expand, but not to retract.

In some embodiments, the drive train may drive a series of mechanisms. The mechanisms may optionally be coupled to the drive in series without sliding or and/or without disengaging. For example, a mechanism may be driven until a stop and then further movement may push the next mechanism in series. For example, the drive train may optionally impel a plunger. Optionally, while the reservoir is sealed, the plunger may drive the reservoir along a direction of motion of the drive train toward an unsealing mechanism. The reservoir may, for example, collide with the unsealing mechanism and be unsealed. After unsealing, the reservoir optionally reaches a stop and ceases moving. Optionally after unsealing the reservoir, further motion of the plunger may drive discharging of the drug.

In some embodiments, one or more mechanisms may be including in the path of the drive train. Optionally, motion of the drive train may activate one or all of the mechanism in its path. In some embodiments, the mechanisms may be activated sequentially and/or simultaneously. Optionally, a first mechanism may be activated until a stopping point at which point further motion of the drive train may activate a second mechanism. For example, a locking mechanism may be located in the path of the drive train. Optionally, while the door is unlocked, a part of the drive train and/or of the plunger and/or of the reservoir may be driven into a locking mechanism, thereby locking a door. For example, after locking the door, subsequent motion of the plunger may drive discharging of the drug. Optionally, motion of the drive train may activate release of a hypodermic needle. Optionally the needle may be released in a direction non-parallel to the motion. For example, the direction of needle release may range between 10° and 90° to the motion. The direction of release of the needle may optionally be substantially parallel to the motion. Optionally, motion of the drive may discharge a flushing fluid before and/or after the medicine.

In some embodiments, the drive train may induce linear movement. For example, a drive train may impel a plunger, which may optionally cause discharging of the drug. Optionally, the linear movement may additionally or alternatively drive a door locking mechanism. For example, the linear movement may cause additionally or alternatively unsealing of a reservoir. For example, the reservoir may be initially sealed with a septum. For example, while a reservoir is sealed, motion of the drive may drive a needle through the septum, unsealing the reservoir. Subsequently to unsealing the reservoir, motion of the drive may cause discharging of the medicine.

For example, the door may be locked when a drive train of the injector contacts a locking mechanism. For example, the drive train may expand, filling a space between a door locking mechanism and a plunger. The drive train may optionally expand backwards towards the locking mechanism. The drive train may eventually contact for example, the locking mechanism causing it to lock. Subsequently, the locking mechanism may, optionally, prevent further backwards motion of the drive train. Further expansion of the drive train may then push the plunger forward into the reservoir.

Optionally, there may be a time delay between activation of the infuser and priming of the infuser. For example, in some embodiments the time delay may range between five seconds and two minutes. For example, the time delay may be the time necessary for a telescoping assembly to fill and empty space. In some embodiments, the length of the empty space may for example be less than 3mm. In some embodiments, the drive train may impart of force ranging for example between 1.0 and 4.0 kg. The length of movement of the drive for priming the pump may range, for example, between 0.4 and 8 mm. In some embodiments, a door lock door may resist an opening force of between 2-8 kg

In some embodiments, the apparatus may be worn by the recipient. Wearing the apparatus may include, for example, attaching a base of the apparatus to the recipient and/or carrying the apparatus attached to clothing of the recipient and/or strapping the apparatus to the recipient. For example, the base of the apparatus may stick to the skin of the recipient (for example via an adhesive).

In some embodiments, there may be a time delay. For example, there may be time delay between activation of an infuser and the activation of a first mechanism. Alternatively or additionally, there may be a time delay between activation and/or stopping of one mechanism and activation of a second mechanism.

In some embodiments, the motion of the drive train may be substantially parallel to the base of the apparatus. For example, the path of motion of the drive train may make an angle between -10° to 10° with an attaching surface of the base.

### Controlled Rate Deliver

In some embodiments, the rate of delivery may be controlled. Optionally a drug may be discharged as a bolus injection and/or continuously and/or at a slow rate and/or at a fast rate. Optionally the rate of discharge may be adjustable. Optionally a sensor may be used to indicate progress of an infusion process. For example, a sensor may measure motion of the drive train. Optionally, processor may user sensor measurement to indicate and/or adjust progress of discharge. For example, based on the rate of motion of the drive train, the processor may compute a rate of drug discharge. For example based on the motion of the drive train the processor may estimate whether a septum has been punctured and/or whether a door has been locked. For example, the rate of motion of the drive train may be adjusted according to results of the measurements.

In some embodiments, the maximum rate of delivery may optionally be between 10 ml/hr and 100ml/hr. Optionally, the total delivered volume may be between 0.5 ml and 20 ml. In some embodiments, the total time of delivery may be between 5 seconds and 20 minutes. Optionally, the total time of delivery may be measured from the beginning of delivery until the end of delivery. Optionally, the beginning of deliver may be measured from the time of activation of the apparatus and/or alternatively from the time of attachment of the apparatus and/or alternatively from the time that delivery of the substance begins. Optionally the end of delivery may be measured at deactivation of the apparatus and/or alternatively at the earliest time after which no more of the substance is delivered and/or alternatively when the apparatus is removed from the recipient. Optionally the drug may be delivered in a single dose and/or at a constant rate.

### Programmable

In some embodiments, a drug pump may be a programmable device. For example, the pump may be capable of controlled delivery of a substance according a preprogrammed schedule and/or rate. Alternatively or additionally, the pump may be a smart device capable of changing a delivery schedule according to commands and/or in reaction to changing conditions. Optionally, the device may be capable of stopping and restarting delivery.

### Sealed for Long Term Storage and Ready to Use

A drug may require strictly prescribed packaging. Legal, health and safety requirements may include very stringent packaging standards. Packages may need to be aseptic, packaging materials may be strictly limited and package geometry may be very precisely stipulated. For example, when a drug is to be stored for a significant period of time (for example more than 24 hours and/or more than a month) standards may be particularly stringent.

### Difficult for Recipient to Unpackage

In some cases, it may be inconvenient for the recipient to prepare a drug for delivery and/or to produce a coordinated force to activate a mechanical trigger. For example, the recipient may include a child and/or the medical procedures may induce fatigue or confusion in the recipient (for example chemotherapy). In some cases, the recipient may have a condition that makes it difficult to perform precise tasks (for example rheumatism and/or Parkinson's disease, and/or partial paralysis of the fingers). More generally, opening packaging and then loading the delivery device may be inconvenient and may increase the probability of exposure to contamination or of error or loss of the drug.

### Ready - examples

It is sometimes desirable to supply a recipient with a delivery device that can be stored. It may also be desirable to supply the delivery device ready to use to deliver a medicine. For example, at a certain time following an outpatient hospital procedure, it may be desirable to deliver a drug to the patient (for example an antidote to chemotherapy agent). Sometimes before a hospital procedure, it may be desirable to deliver a drug to a patient (for example a dye before a radiological diagnostic procedure). A recipient may prefer to receive these drugs from a portable drug pump rather than traveling to a clinic.

### Minimal Involvement of Recipient

In some embodiments, the apparatus may be supplied with a sealed reservoir having an internal space containing a drug. The apparatus may deliver the drug automatically with minimum involvement of the recipient. Optionally, the delivery apparatus may function independently requiring minimal or no cooperation and/or awareness and/or activity of the recipient. For example, a doctor may attach the device to the recipient and the device may take care of delivery of the drug at the proper time without involvement of the recipient. Alternatively or additionally, a recipient may be given a single packaged device and the recipient may be able to take the drug at the required by merely attaching and activating the device.

### Puncture septum vial/Syringe

In some embodiments, a drug reservoir may be sealed by a septum. Optionally, unsealing the reservoir may be by puncturing the septum.

In some embodiments, a drug may be packaged in a vial sealed by a septum. Optionally, the septum may be punctured by inserting a hollow needle through the septum into the vial. In some embodiments, the drug may be packaged in a syringe. Optionally, a needle of the syringe may be sealed by an insertion into a septum. Optionally, unsealing may be achieved by pushing the needle of the syringe through the septum.

In some embodiments, unsealing a package includes piercing a septum. The septum may include, for example, many kinds of seals including caps, stoppers, plugs, diaphragms and/or partitions of various thicknesses. For example, a septum or a seal may be made from plastic, rubber, silicone, a gel, a metal foil, a polymer and/or a combination thereof.

### Portable and/or Low Profile

Optionally, the apparatus may not require conscious carrying by the recipient. Optionally, the apparatus may minimally disturb the recipient. For example, a pump may be small and/or have a low profile. For example, some embodiments of a drug pump may be worn inconspicuously under a recipient's clothing. The height of the apparatus may be less than the square root of the area of the base.

Optionally, the base of the apparatus may be less than 5cm long and/or less than 5cm wide. Optionally, the height of the apparatus may be less than 3 cm. Optionally, the total volume of the apparatus may be less than 100-200ml. Optionally, the mass of the entire apparatus with the substance may be less than 100-200g. Optionally, the capacity of the reservoir for the substance in the apparatus may be between 5-30ml). Optionally, the apparatus may be shock proof and/or waterproof.

The term "reservoir" throughout the specification and claims encompasses any container for a drug, such as but not limited to, a cartridge, vial, syringe, bottle, ampoule and many more, and is not limited to any size or shape. Optionally, the reservoir may be packed and/or stored in the injector. Alternatively or additionally, the reservoir may include a cartridge that is stored separately from the delivery device and inserted into the delivery device at a convenient time.

In some embodiments, the apparatus may be easily disposable (for example in municipal garbage).

### Caveats

### Caveat about needles

In some embodiments, the apparatus is a medicine pump. The invention is some embodiments thereof is not limited to a drug pump, and may be used for any kind of suitable discharge apparatus, not just by needle piercing the patient, but also transdermally (wherein the substance is metered by the apparatus to a transdermal patch), by spray (wherein the substance is metered by apparatus to a spray nozzle), micro needles array and others.

### DETAILED EXEMPLARY EMBODIMENTS

### General Caveat

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Flow Chart of a Method of Delivery of a Drug

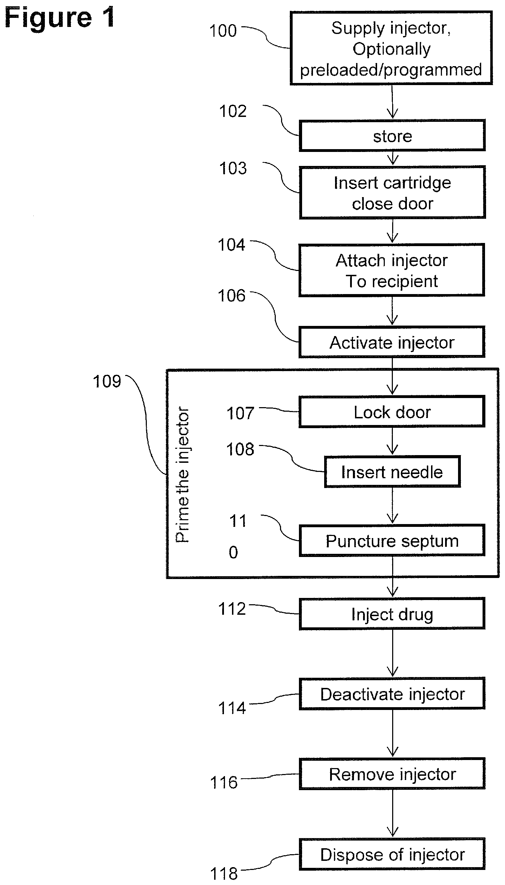

Fig. 1a is a flow chart illustrating an exemplary method of delivering a drug. In the example, it may be preferred that a patient take a drug injection at home and not remain in a medical institution waiting for a nurse to administer the injection. In the exemplary embodiment, the institution is supplied with a portable preloaded automatic apparatus that can be stored until the recipient needs the drug. When the drug is needed, the apparatus is optionally attached to the recipient and/or activated. Optionally, the apparatus takes care of preliminary activities (for example locking a reservoir access door and/or unsealing the drug reservoir and/or inserting a needle into the recipient). The pump then makes the injection and optionally takes care of preparing itself for disposal.

### Supply sloaded Apparatus

In the exemplary embodiment of Fig. 1, the apparatus is optionally supplied 100 to a medical institution. In some embodiments, the injector may be preloaded with the drug and/or preprogrammed. For example, the substance to be injected may be an antidote to a chemotherapy agent to be injected twelve hours after the therapy. Optionally, the antidote is packaged inside the apparatus in a sealed vial. Optionally, the preloaded apparatus may be stored 102 in a medical institution for a time ranging from a few days to a few months before actually being used. Alternatively or additionally, the apparatus may be supplied to the recipient by, for example, a pharmacy and stored by the recipient for a time ranging from a day to a few months until needed. Alternatively or additionally, the sealed vial may be stored separately from the apparatus. Optionally, vial may be loaded into the apparatus by the distributor and/or by a pharmacist and/or by a medical professional (for example a nurse or a doctor) and/or by the recipient himself.

### Program apparatus

In the example of fig. 1, the apparatus is programmed to deliver the entire contents of the reservoir 12 hours after activation in a constant rate injection. Alternatively or additionally, the apparatus may be programmed to deliver a portion of the medicine in a single dose. Optionally the apparatus may be programmed to administer a second dose. Optionally the second dose may be delivered after a fixed time delay and/or the second dose may be delivered on demand of the recipient and/or upon a combination of time delay and command and/or in reaction to some other stimulus. Optionally, preliminary steps may be preprogrammed into the apparatus logic to be carried out before delivering the medicine. Optionally, the drive train may drive a post injection activity such as injecting a flushing fluid after the medicine.

In some embodiments, a cartridge containing the drug may be stored outside of the injector (for example for an injector that is not preloaded). The cartridge may be inserted into a reservoir channel and/or a door to the channel may be closed 103 before use.

### Activate at hospital

In the example of Fig. 1, when the apparatus is needed, the apparatus is optionally attached 104 to a recipient. Attachment may be, for example by an adhesive pad on a base surface of the housing of the apparatus. After attachment, the apparatus is activated 106. Alternatively or additionally, the apparatus may be activated first and then attached to the recipient.

### Unseal then Insert or vice versa

Once activated 106, in some embodiments, the apparatus may perform priming 109 before discharging the drug. For example, in some embodiments, priming may include locking 107 a door. For example, the door may be locked when a drive train contacts a locking mechanism. For example, the drive train may expand, filling a space between a door locking mechanism and a plunger. The drive train may optionally expand backwards towards the locking mechanism. The drive train may eventually contact, for example, the locking mechanism causing it to lock. Subsequently, the locking mechanism may, optionally, prevent further backwards motion of the drive train. Further expansion of the drive train may then push the plunger into the reservoir.

For example, priming **109** may include inserting **108** a needle into the recipient. Optionally, at a preprogrammed injection time, the apparatus may insert **108** a needle into the recipient. Alternatively or additionally, the needle may be inserted **108** into the recipient when the injector is activated. Alternatively or additionally, the needle may be inserted **108** into the recipient before locking **107** the door.

Priming **109** may optionally include unsealing **110** the reservoir. For example, the reservoir may include a vial sealed by a septum and the unsealing **110** include puncturing the septum. Alternatively or additionally, inserting the needle into the recipient may follow and/or precede unsealing the reservoir.

### Delay between unseal and inject

In some embodiments, inserting **108** a needle into the recipient and unsealing **110** the reservoir may open a fluid path between the reservoir and the recipient. Once the fluid path is open, the drug may optionally be discharged **112** into the recipient at a dosage and rate that is optionally controlled by a controller of the injection apparatus.

In some embodiments, discharge **112** of the drug will start quickly after unsealing **110** the reservoir and/or inserting **108** the needle. For example, in some embodiments discharging **112** will start less than 5 minutes after inserting **108** and/or unsealing **110**. In some embodiments, discharging **112** will start less than one minute after inserting **108** and/or unsealing **110**. In some embodiments, there may be a short delay after unsealing **110** the reservoir and/or inserting **108** the needle before discharging **112** the substance. In some embodiments, the delay may range between 30 seconds and 5 minutes.

### Post injection activities chaser/deactivate

In some embodiments, the apparatus may perform post injection tasks. For example, after the drug has been fully or partially discharged **112**, an optional chaser of for example saline solution may be injected. The apparatus may optionally be automatically permanently deactivated **114**. For example, deactivation may include retracting the needle. Once the apparatus is deactivated, it may be removed **116** from the recipient and discarded **118**. Additionally or alternatively, the apparatus may be removed from the recipient and then disabled. For example, upon removal of an injection apparatus, the needle may be retracted and/or a cover may be deployed to protect the needle.

### Single motion

In some embodiments, some or all of insertion **108** of the needle and/or unsealing **110** of the reservoir and/or discharging **112** and/or deactivation **114** of the apparatus may be triggered and/or driven by a single motion drive train. For example, the drive train may include an electric motor and/or a screw and/or a piston. The motion may optionally be continuous. Alternatively or additionally, the injector may be programmed by the distributor and/or medical personnel (for example a nurse, a doctor or a pharmacist) and/or by the recipient.

### An External View of an exemplary Low Profile Drug Injector

### Low Profile

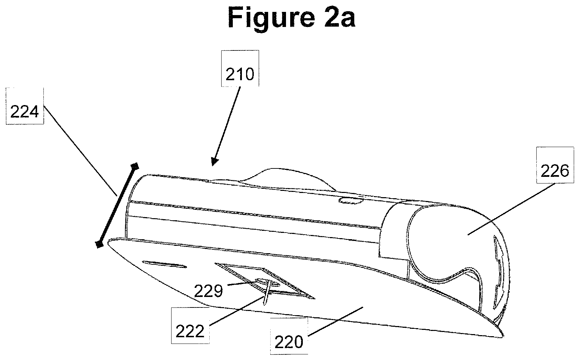

Figure 2a illustrates an external view of an exemplary embodiment **210** of a drug delivery apparatus (a subcutaneous injector apparatus). During use of embodiment **210**, a base surface **220** is stuck to the recipient's skin with an adhesive. A needle **222** extends out a needle opening **229** into a recipient. The drug is discharged via needle **222** into the recipient. Optionally, embodiment **210** is has a large base **220** (for example, the length and width of base **220** is 4 cm by 6 cm) and a low profile (for example, the height **224** of the apparatus is 2 cm). In exemplary embodiment **210**, height **224** of the apparatus is less than half the square root of the surface area of base **220**. In some embodiments, the height may be less the 1.4 times the square root of the surface area of the base. Embodiment **210** may be stabile when stuck to the recipient's skin. Embodiment **210** may be inconspicuous when worn under clothing.

### Tamper proof lock - reuse

A locking door **226** may optionally be provided. In a closed position, door **226** may for example, block a reservoir channel. For example, door **226** may prevent tampering with the drugs inside the injector. It is noted that although embodiment **210** is typically a one-use item, the electronics, batteries and motor and other elements of the system can be used more than once if desired. Locking may occur immediately upon closing door **226** and/or door **226** may be locked after activation of the injector.

### A Schematic Illustration of an Exemplary Drug Pump Having a Septum Puncturing Mechanism

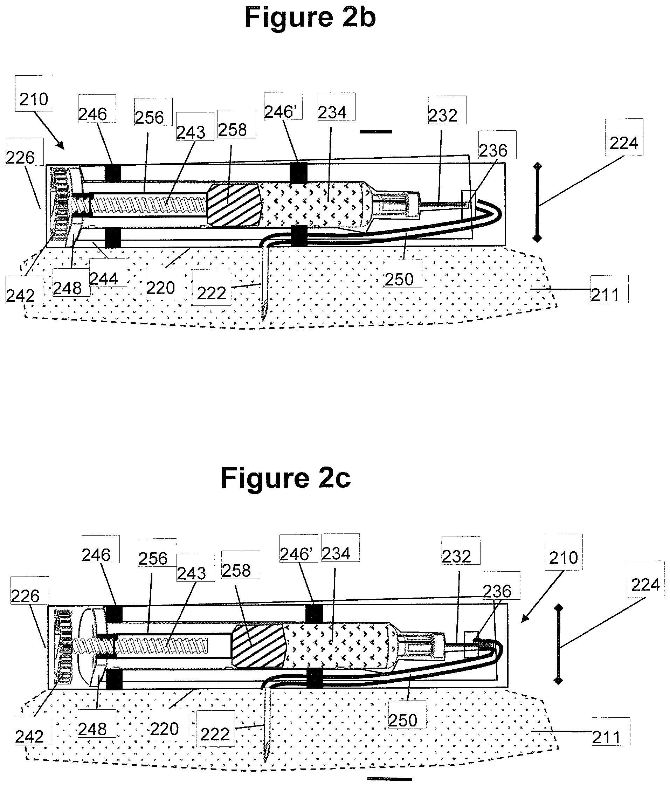

Figs. 2b-d are schematic cutaway side views illustrating exemplary embodiment **210** of a drug delivery apparatus puncturing a septum and delivering a drug.

Fig. 2b illustrates exemplary embodiment **210** in an initial state. In the example, base **220** of the apparatus is attached to the flesh **211** of a recipient. Hypodermic needle **222** protrudes from base **220** of the apparatus into the flesh **211** of the recipient. Optionally, hypodermic needle **222** may be inserted into the recipient by a release mechanism. In some embodiments, needle **222** protrudes perpendicular to base **220**.

In the example, a reservoir **234** is in fluid contact with a hollow needle **232**. Optionally, reservoir **234** and consequently needle **232** are held in place, using low friction pads **246** and **246'.** In the example, in the initial state, reservoir **234** is placed towards the rear (left side) of the apparatus so that there is a gap **244** between a flange **248** of reservoir **234** and pad **246**. In the initial state, needle **232** and consequently reservoir **234** are sealed by a septum **236**.

Some embodiments may include a drive train **256** that translates circular motion of a gear 242 into translational motion. For example, drive train **256** may include a mechanical actuator, which causes backward and/or forward motion along a single path, for example a straight line. For example, drive train **256** may include a telescoping assembly. In the exemplary embodiment, activating a motor supplies a torque to turn gear **242** and screw **243**. Optionally, the housing of the apparatus prevents translation motion of gear **242** and screw **243**.

In some embodiments, rotation of screw **243** causes drive train **256** to telescope. While reservoir **234** is sealed, drive train **256** pushes reservoir **234** along a linear path of motion parallel to base **220** of the apparatus. Needle **232** may optionally be located along the path of motion of drive train **256**. Needle **232** may be directed along the path of motion of drive train **256**. Needle **232** may optionally be pointed toward septum **236**. Expansion of drive train **256** advances needle **232** through septum **236**. Eventually, needle **232** pierces septum **236**, unsealing reservoir **234** as shown, for example, in Fig. 2c.

In embodiment **210**, the motion of drive train **256** and/or reservoir **234** may be parallel to the base of the apparatus. In embodiment **210**, the path of motion of a plunger **258** is parallel to the base of the apparatus. In embodiment **210,** the long axis of reservoir **234** is parallel to the base of the apparatus. The Length of base **220** of exemplary embodiment **210** is longer than the height **224** of the apparatus. In some embodiments, the long axis of and/or the pathway followed by reservoir **234** may be directed non-parallel to and/or orthogonal to hypodermic needle **222**. In Fig. 2c, the exemplary embodiment is shown with needle **232** puncturing septum **236.** In the example, flange **248** is in contact with pad **246** stopping further forward movement of reservoir **234.**

In some embodiments, puncturing septum **236** creates a fluid pathway from reservoir **234** through hollow needle **232** and through a canal **250** and through hypodermic needle **222** to flesh **211** of the recipient.

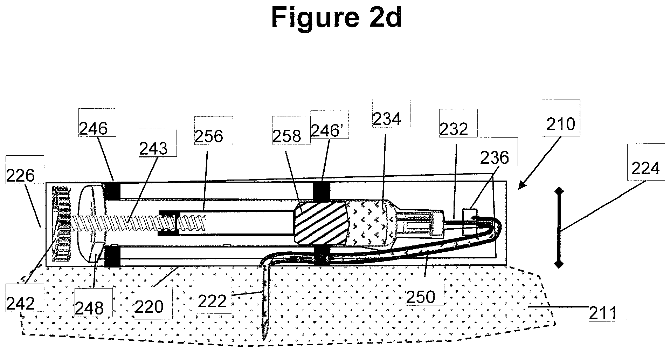

In the exemplary configuration of Fig. 2c, further, drive train **256** advances a plunger **258** inside of reservoir **234** as illustrated, for example, in Fig. 2d. Advancement of plunger **258** discharges the drug out of needle **232,** canal **250** and needle **222** into flesh **211** of the recipient.

### An Exemplary Syringe Reservoir with a Needle for Puncturing a Septum

Figs. 3, 3', 4 and 4' illustrate another exemplary embodiment **310** of a drug delivery apparatus. In embodiment **310,** a syringe needle **332** is mounted to a reservoir **334**. In figs. 3 and 3' needle **332** is shown sealed by an optional septum **336**. In Figs. 4 and 4' needle **332** is shown puncturing septum **336** to unseal reservoir **334**.

Figs. 3 and 4 illustrate an optional programmable controller **328** capable of directing an optional actuator **330** for powering discharging of a substance. In addition, figs. 3, 3', 4 and 4' illustrate how actuator **330** may optionally drive puncturing of septum **336** and/or trigger insertion of a needle into the recipient.

### Programmable

In some embodiments, controller **328** may be a programmable electronic processor. Controller **328** may optionally include a memory.

In some embodiments, actuator **330** may include a direct current (DC) electric motor. Power for controller **328** and actuator **330** may be supplied by a power supply **338** (for example batteries). Controller **328** may optionally direct actuator **330** by pulse width modulation (PWM). Alternatively or additionally, actuator **330** may include a brushless DC servo (for example a stepper motor) directed by controller **328**. In some embodiments, the rate of rotation of actuator **330** may be adjustable. The rate of discharge of the drug may correspond to the rate of rotation of the actuator **330**. Optionally, rate of rotation of the actuator **330** may be adjusted by programming controller **328** and/or by a user interface (for example a dial and/or button) on the apparatus.

### Detect fault e.g. failure to move

Embodiment **310** includes an optimal rotation sensor **340**. In some embodiments rotation sensor **340** may include an optical sensor that detects movements of a paddle. Alternatively or additionally, rotation sensor may include a Hall Effect sensor or the like. Optionally, if actuator **330** fails to rotate and/or rotates too slowly upon application of power, controller **328** may notify the recipient of a malfunction. Other malfunctions that may be detected include not enough voltage, not enough current, too much current, and/or too fast rotation.

### Needle mounted to reservoir sealed by septum

In Figs. 3 and 3', a drug reservoir **334** is shown in a sealed configuration. Reservoir **334** is held in place for example by low friction pads **346** and **346'**. In the sealed configuration, needle **332** is optionally sealed by septum **336,** for example, as illustrated in Fig. 3'.
In some embodiments, when reservoir **334** is in the sealed configuration, a gap **344** exists between a flange **348** of reservoir **334** and friction pad **346**.

### Pump mechanism puncture septum

In some embodiments, a transmission including gears **342** which connect actuator **330** to a telescoping drive train (for example see drive train **456** fig. 4) located inside reservoir **334**. A more detailed view of an exemplary embodiment of a drive train **556** is shown in figs. 5 and 6. For example, drive train **556** may include a telescoping assembly. For example, the telescoping assembly may include one or more concentric screw threads and/or a gear. For example, the telescoping assembly may convert rotational motion into linear expansion and/or contraction of the assembly. Alternatively or additionally, a drive train may include a linear actuator. In exemplary embodiment **310,** the transmission includes a gear, but alternatively or additionally, it could include another coupling element for transmitting rotary motion, such as a friction wheel. Optionally rotation of actuator **330** drives expansion of telescoping drive train **456**. Alternatively or additionally, the drive mechanism may include an expanding gas chamber. For example, expanding gas may be produced by an electrolytic cell and/or by a chemical reaction. Alternatively or additionally, the reservoir may have flexible walls. Optionally, discharge may include deforming and/or squeezing the reservoir.

In embodiment **310**, while reservoir **334** is in the sealed configuration of Figs. 3 and 3', expansion of drive train **456** drives reservoir **334** and needle **332** forward. Optionally, forward motion stops when flange **348** contacts friction pad **346**, which acts as a stop along the path of motion of reservoir **334**.

In some embodiments, forward movement of reservoir **334** pushes needle **332** through septum **336** putting the apparatus into the discharge configuration, for example, as illustrated in fig. 4. In the discharge configuration, a gap **444** has formed behind flange **348**. The rear end of telescoping drive train **456** can be seen in gap **444**. In the discharge configuration, needle **332** has fully punctured septum **336**, unsealing reservoir **334**. A fluid path exists from reservoir **334** through needle **332** to a canal **350.**

In embodiment **310**, the path of motion of reservoir **334** is parallel to the base of the apparatus. In embodiment **310**, the path of motion of drive train **456** is parallel to the base of the apparatus. In embodiment **310,** the long axis of reservoir **334** is parallel to the base of the apparatus. The base of the apparatus is longer than the height of the apparatus.

### Insert needle before/after septum

Embodiment **310** includes an optional trigger arm **352** for triggering a needle release **354**. As reservoir **334** moves forward it displaces arm **352** causing needle release **354** to insert a hypodermic needle (not shown) into the recipient. A tube (not shown) supplies a fluid pathway from canal **350** to the hypodermic needle. In the discharge configuration, there is a fluid pathway from reservoir **334** to the recipient. Optionally, the hypodermic needle may be inserted into the recipient either before or after completing puncturing of septum **336**. Alternatively or additionally, controller **328** may directly control needle release **354**. Optionally, the direction of release of the needle may be non-parallel and/or orthogonal to the direction of movement of drive train **456**.

### Measure flow/ know status

In the example of embodiment **310,** telescoping drive train **456** includes a threaded member to convert rotational motion of actuator **330** into expansion and translational motion of the drive train **456.** Optionally, in embodiment **310** rotation of sensor **340** may be directly proportional translational movement. In embodiment **310**, measurements of rotation can be used to sense the position of the translational mechanism. By means of measurements of the rotation of actuator **330,** controller **328** optionally tracks the location of telescoping drive train **456** and/or of reservoir **334** and/or of needle **332.** Optionally the rate of discharge of the drug may be detected by measuring a rate of rotation of sensor **340**. Optionally, the state of the septum **336** (for example whether drive train **456** has advanced far enough to puncture septum **336**) and/or the cumulative volume of medicine discharged may be sensed based on the cumulative number of revolutions of rotation sensor **340.**

### Discharge

In embodiments **310**, when reservoir **334** is in the discharge configuration, friction pad **346** prevents further forward motion of reservoir **334**. As stated above, in the discharge configuration, there is a fluid flow path from reservoir **334** into the recipient. Further expansion of the drive train **456** impels a plunger into reservoir **334** discharging the contents of reservoir **334** out needle **332** into canal **350** and out canal **350** through the hypodermic needle into the recipient.

### A Reservoir where a Hollow Needle Is Inserted into a Vial through a Septum

Figure 5 is a cutaway illustration of an exemplary embodiment **510** of a drug delivery apparatus. In embodiment **510**, a reservoir is sealed by a septum. Optionally, the septum is punctured by driving the reservoir toward a hollow needle until the point of the needle punctures the septum. Upon puncturing the septum, the point of the needle enters the reservoir. Subsequently, the hollow of the needle provides for fluid communication between the reservoir and the external environment.

Embodiment **510** includes a reservoir **534** sealed by a septum **536**. Optionally, reservoir **534** may be placed a bit away from a hollow needle **532** in a sealed configuration (For example, as illustrated in Fig. 5). Reservoir **534** may be held in place, for example, using low friction pads **546**, with needle **532** against and/or partially inserted into and/or adjacent to septum **536**.

In embodiment **510**, activating a motor **530** may cause a drive train **556** to telescope. Rearward movement of drive train **556** may optionally be restrained by a hub **557** mounted on a door **526**. Telescoping drive train **556** may push a plunger **558** into reservoir **534**. In the example of embodiment **510**, telescoping drive train **556** includes concentrically mounted threaded members to convert rotational motion of an actuator, motor **530**, into expansion of drive train **556** and translation of plunger **558.** Because reservoir **534** is sealed, translation of plunger **558** advances reservoir **534** against needle **532**, puncturing septum **536** (as shown, for example, if Fig. 6). Optionally, reservoir **534** translates forward until a cap **548** of reservoir **534** crosses a gap **544** and contacts a stop **549**.

Puncturing septum **536** creates a fluid flow path between reservoir **534** and the recipient's body. The flow path allows further translation of plunger **558** to produce fluid flow out needle **532** and through a tube (not shown) into a hypodermic needle (not shown) and into the recipient.

Embodiment **510** includes further optional components, for example, batteries **538**, and a needle release **554**, and a rotation sensor **540**, and a controller **528**, and a transmission **542**.

### A Septum Preserving Aseptic Conditions of a Hypodermic Needle

Figs. 7a and 7b illustrate perspective views of an exemplary embodiment of a needle insertion mechanism **754** with a needle opening septum **764**. Needle opening septum 764, seals a needle opening, preserving aseptic conditions inside an injector (as illustrated, for example, in Fig. 7c).

In some embodiments, when the apparatus is attached to a recipient, when needle **722** is released, it may optionally pass through needle opening septum **764** (as illustrated, for example, in Fig. 7b) and be inserted into the flesh of the recipient.

In some embodiments, prior to release, needle **722** may be partially inserted into needle opening septum **764** (for example as illustrated in Fig. 7a). While partially inserted, the opening at the tip of needle **722** may be sealed by needle opening septum **764**. Needle **722** may be in fluid communication with a drug reservoir. Sealing needle **722** may seal the reservoir. Alternatively or additionally, prior to release, a hypodermic needle **722'** may be held clear of a needle opening septum **764',** as illustrated, for example in Fig. 7c).

In the example of Figs. 7a,b a motor **730** rotates a shaft **760**. Rotation of shaft **760** optionally causes a threaded arrester **762** to slide out of an opening in insertion mechanism **754** releasing needle **722.** In some embodiments, motor **730** may also trigger unsealing a reservoir and discharging of a drug (as illustrated, for example, above). An optional rotation sensor includes sensors **741**, which sense revolutions of a paddle **740.**

In some embodiments, needle opening septum **764'** may be fixed to the housing of the injection apparatus. For example, Figs. 7c and 7d are cutaway perspective illustrations of an exemplary embodiment where needle opening septum **764'** is connected to a base **720** of the housing of the injection apparatus. Fig. 7c illustrates the exemplary embodiment prior to release of a needle **722'** by a release **754'**. In the illustration, needle opening **729'** is sealed by needle opening septum **764'**. Fig. 7d illustrates the exemplary embodiment after release of needle **722'** and after needle **722'** punctures needle opening septum **764'.**

Fig. 8 is a flow chart illustration of an exemplary embodiment of a method for delivering a drug to a recipient. In some embodiments, an injector apparatus may be supplied **870** in a sealed state. Optionally in the sealed state, an internal space of the injector may be preserved **872** in an aseptic state by a septum and/or by a sealed door. Optionally, a hypodermic needle may be stored in the aseptic internal space of the injector. Alternatively or additionally, an injector and/or the cartridge may be supplied in a sealed aseptic blister. In the blister, the injector may optionally be supplied in an open and/or closed state.

In some embodiments, the apparatus may be attached **874** to the recipient while still in the sealed state. For example, a base of the apparatus may be stuck to the recipient using an adhesive.

In some embodiments, there may be a release mechanism. The mechanism may release **876** the needle. For example, the needle may be released **876** after attachment **874** of the apparatus to the recipient. Optionally upon release **876**, the needle may puncture **878** the septum. In some embodiments, the needle may continue through the septum, through a needle opening in the base of the apparatus and be inserted **880** into the recipient.

Once the needle is inside the recipient, a drug may optionally be delivered **882** through the needle into the recipient.

### Door Locking Mechanisms

Figs. 9A and 9A illustrate respective wide and close up cutaway views of an exemplary embodiment of a mechanism locking a door of an infuser. For example, a door may include a latch that may engage catch in the housing of the infuser, locking the door closed. Optionally, the door and/or the latch may be biased away from the catch and/or play in the door may allow the latch to be disengaged from the catch. In some embodiments, activation of the drive train may drive the latch into engagement with the catch, locking the door.

In some embodiments, the drive train may include a telescoping assembly. The telescoping assembly may optionally be attached at its front end to a plunger in a medicine reservoir. Behind the telescoping assembly, there may optionally be some free space and/or a door. As the telescoping assembly expands, it may fill the free space and then optionally push backwards against the door and forward against the reservoir. In some embodiments, pushing the door backwards may cause engagement of the latch and the catch. Further rearward movement may optionally be restrained by the door and/or the latch. In some embodiments, pushing forward against the reservoir may cause unsealing of the reservoir. Forward movement of the reservoir may optionally be restrained by contact between a flange and/or a front end of the reservoir with the housing. Further, expansion of the telescoping assembly may, in some embodiments, force the plunger into the reservoir, discharging the medicine.

In some embodiments, a latching mechanism may include, a hub **957** and/or a latch **986** and/or a catch **984**. Latch **986** may optionally be configured to engage catch **984** holding door **986** immobile with respect to the housing of the infuser. Door **926** may optionally be configured to swing upward and/or downward around a pivot. Closing the door may, for example, block access to a reservoir channel. The mounting of door **926** may leave slack allowing forward and backward movement of the door engaging and/or disengaging latch **986** with catch **984**.
In some embodiments, activating the apparatus may drive a rear end of a drive train **956** into hub **957**. This may, optionally, drive door **926** backwards taking up the slack in its movement. Driving door **926** backwards may, for example, force latch **986** into engagement with catch **984** thereby preventing door **926** from swinging upward. This may, optionally, permanently lock door **926**.

In some embodiments, the slack in the door may range between, for example, 0.1 and 3mm.

Figs. 10A-D illustrate a second exemplary embodiment of a locking mechanism for an infuser. The locking mechanism of Figs. 10A-D may optionally include a temporary latch and/or a permanent latch. Upon closing a door, the temporary latch may optionally hold the door closed. In some embodiments, a user may be able to disengage the temporary latch. For example by pushing sideways on a tab, may release the temporary latch and open the door. Upon activating the infuser, a drive train may optionally drive engagement of a permanent locking mechanism. For example, the permanent locking mechanism may prevent disengagement of the latch.

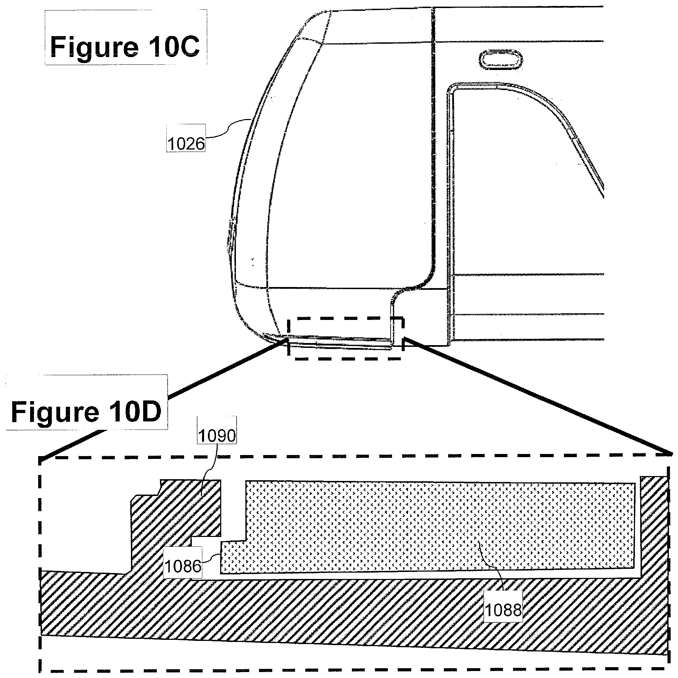

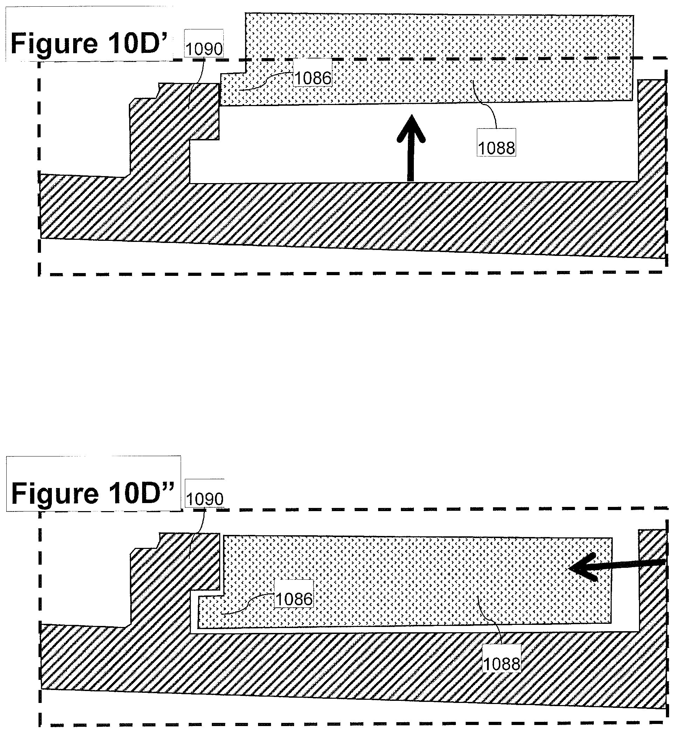

Figs. 10A,B,B' illustrate a cutaway side view of an exemplary embodiment of a locking mechanism. Fig. 10B shows a close-up cutaway view of the exemplary locking mechanism in a permanently locked state. Fig. 10B' shows a close-up cutaway view of the exemplary locking mechanism in a temporarily locked state. Fig. 10C illustrates an overhead view of the exemplary infuser. Fig. 10D shows a cutaway overhead view of the infuser in a temporarily locked state. Fig. 10D' shows a cutaway overhead view of the infuser in a released state. Fig. 10D" shows a cutaway overhead view of the infuser in a permanently locked state.

In some embodiments, the locking mechanism may include a door **1026** and/or a permanent latch **1086** and/or a temporary latch **1088** and/or a catch **1084a** for temporary latch **1088** and/or a catch **1084b** for permanent latch **1086**.

In the exemplary embodiment of Figs. 10A-D, door **1026** opens by swinging upward. In the locked state, (for example as illustrated in Figs. 10A,B,B' and D) catches **1084a** and/or **1084b** block respective latches **1088** and/or **1086** from moving upward, thereby preventing opening of door **1026**.

In some embodiments, a user may a temporary latch from its latched state (for example the latched state of Figs. 10B',D). For example, temporary latch **1088** may be released by pushing inward on a tab on the side of door **1026** (for example as illustrated by the arrow in Fig. 10D'). Pushing the side inward may, for example, move latches **1086** and/or **1088** inward away from catches **1084a,b** freeing door **1026** to swing upward.

In some embodiments, activating a drive train **1056** (see Fig. 10A) causes drive train 1056 to push backwards against door **1026** (for example as illustrated by the arrow of Fig. 10A,B,D"). Backwards motion of door **1026** may (as illustrated by the arrows in Figs. 10B and 10D") drive backwards, permanent latch **1086**. In the backwards position latch **1086** is trapped by a block **1090.** Block **1090** prevents latches **1086** and **1088** from moving sidewardly inward, preventing release of door **1026.**

Figs. 11A,B illustrate another alternate exemplary embodiment of a locking mechanism in an unlocked and locked position respectively. In some embodiments, a bolt **1192** may optionally be located along the path of motion of a drive train (not shown). Bolt **1192** may be pushed by the drive train into a hole **1194**. Once inside hole **1194** bolt **1192** prevents a door **1126** from moving with respect to a holw **1194** thereby locking door **1126** in a closed state. Fig. 12A-C illustrate an exemplary schematic embodiment of an infuser having a preliminary door lock and a preliminary unsealing mechanism. In the exemplary embodiment, the infuser includes a reservoir in a chamber. Access to the chamber is controlled by a door. The cartridge optionally includes a mechanical drive train, which can expand linearly. The door and/or the locking mechanism and/or the reservoir and/or a plunger and/or a seal and/or an unsealing mechanism may optionally be lined up along a path of motion of the drive train. Preliminary expansion of the drive train may prime the apparatus. For example, expansion of the drive train may drive the cartridge against the door locking mechanism thereby locking the door and/or expansion of the drive train may drive the cartridge against a seal breaking mechanism thereby unsealing the reservoir. After unsealing the reservoir and/or locking the door, further movement of the drive train may optionally drive the plunger into the reservoir dispensing the medicine.

Fig 12A shows the exemplary embodiment of an infuser in an unprimed configuration. In the unprimed configuration a telescoping drive train **1256** is in a collapsed state. The user closes an access door **1226** and activates drive train **1256.**

As drive train **1256** expands, it optionally pushes a rear end of drive train **1256**. Drive train **1256** may, for example expand rearward. A locking mechanism including a rotating bolt **1292** is optionally situated along the path of motion of the drive train **1256.** As drive train **1256** expands it optionally contacts bolt **1292** optionally rotating bolt **1292** to lock door **1226** closed (as can be seen for example in Fig. 12B). Optionally, if door **1226** is open a warning may be issued and/or the infuser may not function. Optionally drive train **1256** continues to expand pushing the rear end of drive train **1256** backwards until it optionally contacts the locked door **1226.** Door **1226** may optionally stop rearward progress.

With the rear end of drive train **1256** blocked by door **1226,** further, expansion of drive train **1256** optionally pushes the front end of drive train **1256** forward. A plunger **1258** is optionally situated along the path of motion of drive train **1256.** As long as reservoir **1234** is sealed, plunger **1258** does not enter reservoir **1234.** As long as reservoir **1234** is sealed, plunger **1258** pushes reservoir **1234** forward. A seal **1236** of reservoir **1234** is optionally pushed forward along a path of motion or drive train **1256** until it contacts an unsealing mechanism **1232** Unsealing mechanism, **1232** may optionally be situated along the line of motion of drive train **1256**. For example, seal **1236** may include a septum and unsealing mechanism **1232** may include a hollow needle directed along the path of motion and pointed toward the septum. For example, drive train **1256** may drive the needle through the septum breaking the seal and/or creating a path for discharging the medicine (for example through the hollow of the needle). Locking the door and/or breaking the seal may optionally place the infuser into a primed state (for example as illustrated in Fig. 12B).

Once the infuser is primed, further expansion of drive train **1256** may drive plunger **1258** into reservoir **1234**, discharging the medicine.

### General

It is expected that during the life of a patent maturing from this application many relevant technologies will be developed and the scope of the terms is intended to include all such new technologies *a priori.*

As used herein the term "about" refers to ± 10 %.

The terms "comprises", "comprising", "includes", "including", "having" and their conjugates mean "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements. Although the invention has been described in conjunction with specific embodiments thereof, it is evident that many alternatives, modifications and variations will be apparent to those skilled in the art. Accordingly, it is intended to embrace all such alternatives, modifications and variations. In addition, citation or identification of any reference in this application shall not be construed as an admission that such reference is available as prior art to the present invention. To the extent that section headings are used, they should not be construed as necessarily limiting.

## Claims

1. An apparatus for delivering a drug to a recipient comprising:
an attaching base (220) for attachment to the recipient;
a single motion mechanical drive train (242, 243, 256; 330); and
a reservoir (234, 334) containing said drug;
**characterized in that**:
a puncturing mechanism (232, 332) including a hollow needle (232, 332) is in fluid communication with the reservoir (234, 334), the hollow needle (232, 332) is being partially inserted into a septum (236, 336) in an initial, sealed configuration of the apparatus, thereby sealing the hollow needle (232, 332) and the reservoir (234, 334), the mechanical drive train (242, 243, 256; 330) being configured to drive the reservoir (234, 334) and the hollow needle (232, 332) forward to push the hollow needle (232, 332) fully through said septum (236, 336), to unseal the hollow needle (232, 332) and the reservoir (234, 334), and put the apparatus in a discharge configuration, and
further motion of said mechanical drive train (242, 243, 256; 330) being configured to advance a plunger (258) into said reservoir (234, 334), thereby discharging the drug from said reservoir (234, 334).

2. The apparatus of claim 1, further comprising:
a controlled rate power source driving said drive train (242, 243, 256; 330).

3. The apparatus of claim 2, where said controlled rate power source includes at least one element selected from the group consisting of a direct current motor, a stepper motor, and a linear actuator.

4. The apparatus of claim 1, wherein said motion is substantially parallel to said attaching base (220).

5. The apparatus of claim 1, further comprising:
a hypodermic needle (222, 722), and
a needle release (354), for inserting said hypodermic needle (222, 722) into the recipient, said needle release (354) being activated by said motion.

6. The apparatus of claim 4, further comprising:
a hypodermic needle (222, 722), and
a needle release (354), for inserting said hypodermic needle (222, 722) into the recipient, a direction of said needle release (354) being non-parallel the path of motion.

7. The apparatus of claim 5, wherein a needle opening septum (764) is located in a needle opening of the apparatus such that upon release said hypodermic needle (222, 722) punctures said needle opening septum (764) before being inserted into the recipient.

## Patentansprüche

1. Vorrichtung zur Abgabe eines Arzneimittels an einen Empfänger, umfassend:
eine Befestigungsbasis (220) zur Befestigung an dem Empfänger;
einen mechanischen Einzelbewegungsantriebsstrang (242, 243, 256; 330); und
einen Vorratsbehälter (234, 334), der das Arzneimittel enthält;
**dadurch gekennzeichnet, dass**:
ein Punktionsmechanismus (232, 332), der eine Hohlnadel (232, 332) umfasst, in Fluidverbindung mit dem Vorratsbehälter (234, 334) steht, die Hohlnadel (232, 332) in einer anfänglichen, abgedichteten Konfiguration der Vorrichtung teilweise in ein Septum (236, 336) eingesetzt ist, wodurch die Hohlnadel (232, 332) und der Vorratsbehälter (234, 334) abgedichtet werden, der mechanische Antriebsstrang (242, 243, 256; 330) konfiguriert ist, um den Vorratsbehälter (234, 334) und die Hohlnadel (232, 332) nach vorne zu treiben, um die Hohlnadel (232, 332) vollständig durch das Septum (236, 336) zu drücken, um die Abdichtung der Hohlnadel (232, 332) und des Vorratsbehälters (234, 334) zu entfernen und um die Vorrichtung in eine Ausstoßkonfiguration zu versetzen, und
eine weitere Bewegung des mechanischen Antriebsstrangs (242, 243, 256; 330) konfiguriert ist, um einen Kolben (258) in den Vorratsbehälter (234, 334) vorzuschieben, wodurch das Arzneimittel aus dem Vorratsbehälter (234, 334) abgegeben wird.

2. Vorrichtung nach Anspruch 1, ferner umfassend:
eine Stromquelle mit gesteuerter Rate, die den Antriebsstrang (242, 243, 256; 330) antreibt.

3. Vorrichtung nach Anspruch 2, wobei die Stromquelle mit gesteuerter Rate mindestens ein Element beinhaltet, das aus der Gruppe ausgewählt ist, bestehend aus einem Gleichstrommotor, einem Schrittmotor und einem Linearstellglied.

4. Vorrichtung nach Anspruch 1, wobei die Bewegung im Wesentlichen parallel zu der Befestigungsbasis (220) verläuft.

5. Vorrichtung nach Anspruch 1, ferner umfassend:
eine hypodermische Nadel (222, 722) und
eine Nadelfreigabe (354), um die hypodermische Nadel (222, 722) in den Empfänger einzuführen, wobei die Nadelfreigabe (354) durch die Bewegung aktiviert wird.

6. Vorrichtung nach Anspruch 4, ferner umfassend:
eine hypodermische Nadel (222, 722) und
eine Nadelfreigabe (354), um die hypodermische Nadel (222, 722) in den Empfänger einzuführen, wobei eine Richtung der Nadelfreigabe (354) nicht parallel zu dem Bewegungspfad ist.

7. Vorrichtung nach Anspruch 5, wobei ein Nadelöffnungs-Septum (764) in einer Nadelöffnung der Vorrichtung derart angeordnet ist, dass bei dem Freigeben die hypodermische Nadel (222, 722) das Nadelöffnungs-Septum (764) durchbohrt, bevor sie in den Empfänger eingeführt wird.

## Revendications

1. Appareil destiné à administrer un médicament à un récipient, comprenant :
une base de fixation (220) pour fixation au récipient ;
une transmission mécanique à mouvement unique (242, 243, 256 ; 330) ; et
un réservoir (234, 334) contenant ledit médicament ;
**caractérisé en ce que** :
un mécanisme de perforation (232, 332) comprenant une aiguille creuse (232, 332) est en communication fluidique avec le réservoir (234, 334), l'aiguille creuse (232, 332) est partiellement insérée dans un septum (236, 336) dans une configuration initiale étanche de l'appareil, fermant ainsi de manière étanche l'aiguille creuse (232, 332) et le réservoir (234, 334), la transmission mécanique (242, 243, 256 ; 330) étant configurée pour faire avancer le réservoir (234, 334) et l'aiguille creuse (232, 332) afin de pousser l'aiguille creuse (232, 332) entièrement à travers le septum (236, 336), ouvrir l'aiguille creuse (232, 332) et le réservoir (234, 334) et placer l'appareil dans une configuration de décharge, et
le mouvement ultérieur de ladite transmission mécanique (242, 243, 256 ; 330) étant configuré pour faire avancer un piston plongeur (258) dans ledit réservoir (234, 334), évacuant ainsi le médicament dudit réservoir (234, 334).

2. Appareil selon la revendication 1, comprenant, en outre :
une source d'énergie à vitesse régulée entraînant ladite transmission (242, 243, 256;330).

3. Appareil selon la revendication 2, dans lequel ladite source d'énergie à vitesse régulée comprend au moins un élément choisi dans le groupe consistant en un moteur à courant continu, un moteur pas à pas et un actionneur linéaire.

4. Appareil selon la revendication 1, dans lequel ledit mouvement est sensiblement parallèle à ladite base de fixation (220).

5. Appareil selon la revendication 1, comprenant, en outre:
une aiguille hypodermique (222, 722), et une libération d'aiguille (354), pour insérer ladite aiguille hypodermique (222, 722) dans le récipient, ladite libération d'aiguille (354) étant activée par ledit mouvement.

6. Appareil selon la revendication 1, comprenant, en outre :
une aiguille hypodermique (222, 722), et
une libération d'aiguille (354), pour insérer ladite aiguille hypodermique (222, 722) dans le récipient, une direction de ladite libération d'aiguille (354) étant non parallèle à la trajectoire du mouvement.

7. Appareil selon la revendication 5,
dans lequel un septum d'ouverture d'aiguille (764) est situé dans une ouverture d'aiguille de l'appareil de façon telle que, lors de la libération, ladite aiguille hypodermique (222, 722) perfore ledit septum d'ouverture d'aiguille (764) avant d'être insérée dans le récipient.
